(19) Europäisches Patentamt
European Patent Office
Office européen des brevets

(11) **EP 3 550 504 A1**

(12) **EUROPEAN PATENT APPLICATION**
published in accordance with Art. 153(4) EPC

(43) Date of publication:
**09.10.2019 Bulletin 2019/41**

(51) Int Cl.:
**G06Q 50/22** (2018.01) **G06Q 50/10** (2012.01)
**A63B 24/00** (2006.01)

(21) Application number: **17876292.8**

(86) International application number:
**PCT/KR2017/011197**

(22) Date of filing: **11.10.2017**

(87) International publication number:
**WO 2018/101597 (07.06.2018 Gazette 2018/23)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **29.11.2016 KR 20160160069**

(71) Applicant: **NEOFECT Co., Ltd.
Yongin-si, Gyeonggi-do 16890 (KR)**

(72) Inventors:
• **YI, Hyo Seok**
**Yongin-si, Gyeonggi-do 16923 (KR)**
• **KIM, Hyun Soo**
**Seoul 06523 (KR)**
• **SONG, Ho Yeong**
**Yongin-si, Gyeonggi-do 16875 (KR)**

(74) Representative: **Zardi, Marco
M. Zardi & Co. SA
Via Pioda 6
6900 Lugano (CH)**

(54) **EXTRACTION ORDER DETERMINING METHOD USING COMPUTER, AND PROGRAM**

(57) Disclosed are a method and a program for determining a sequence of extractions by using a computer. The method includes an operation of receiving a first selection ratio for a plurality of selection targets by the computer (S200) (a first selection ratio receiving operation), an operation of determining speeds of first extraction counters for the selection targets (S400) (a first extraction counter speed determining operation), and an operation of repeating a process of extracting a specific selection target from the plurality of selection targets by driving the first extraction counters for the selection targets (S600).

【FIG. 1】

Receive first selection ratio for plurality of selection targets by the computer [S200]

↓

Determine speeds of first extraction counters for selection targets [S400]

↓

Repeat process of extracting specific selection target from plurality of selection targets by driving first extraction counters for selection targets [S600]

## Description

### BACKGROUND

Embodiments of the inventive concept described herein relate to a method and a program for determining a sequence of extractions by using a computer, and more particularly, relate to a method and a program for generating a sequence in which a plurality of selection targets have a specific selection ratio.

Rehabilitation training or weight training includes several selection targets that are to be performed, and a sequence for performing the selection targets is necessary. When several selection targets are performed at the same ratio, the selection targets have to be performed sequentially. However, if there is a difference between the ratios at which the selection targets are performed, the performance ratio cannot be achieved when they are performed sequentially. If the performance ratio that is suitable for the user is not reflected on the sequence of performance, only a specific body portion may be developed or rehabilitation of a body portion of a low rehabilitation level cannot be properly performed.

Because the user performs an exercise together with a rehabilitation therapist or a personal trainer when rehabilitation training or weight training is performed, the rehabilitation therapist or the personal trainer may set a training sequence that is suitable for the patient or the exerciser but there is a temporal, spatial, and cost restrictions in performing rehabilitation training together with the rehabilitation therapist or the personal trainer.

Accordingly, in order that the user may perform an exercise in a sequence that provides a suitable rehabilitation or exercise effect without an assistant such as a rehabilitation therapist, a method for generating a performance sequence having a selection ratio (that is, a performance ratio) that is suitable for the user is necessary.

### SUMMARY

Embodiments of the inventive concept provide a method and a program for determining a sequence of extractions by using a computer, by which a performance sequence of selection targets is set as a plurality of selection targets are extracted such that the plurality of selection targets are uniformly distributed while a specific selection ratio is maintained as a whole.

The technical objects of the inventive concept are not limited to the above-mentioned ones, and the other unmentioned technical objects will become apparent to those skilled in the art from the following description.

In accordance with an aspect of the inventive concept, there is provided a method for determining a sequence of extractions by using a computer, the method including a first selection ratio receiving operation of receiving a first selection ratio for a plurality of selection targets, a first extraction counter speed determining operation of determining speeds of first extraction counters for the selection targets, wherein the first extraction counters are set for the selection targets and wherein the speeds are proportional to the first selection ratio, a selection target extracting operation of, if a first extraction counter for a specific selection target reaches a reference value as the counter value is increased at a speed determined for the first extraction counter, extracting a selection target, the counter value of which reaches the reference value, a first extraction counter initializing operation of initializing the first extraction counter of the extracted selection target, and an operation of repeating the selection target extracting operation and the first extraction counter initializing operation.

The method may include a first selection ratio receiving operation of receiving a first selection ratio for a plurality of selection targets, a first extraction counter speed determining operation of determining speeds of first extraction counters for the selection targets, wherein the first extraction counters are set for the selection targets and wherein the speeds are proportional to the first selection ratio, a selection target extracting operation of, as the counter values at speeds determined for the respective first extraction counters are increased, extracting a selection target, of which the counter value of the first extraction counter is maximal, from the plurality of selection targets, a first extraction counter correcting operation of calculating a first calculation value by subtracting a sum of the speeds for the first extraction counters for the selection targets from the counter value of the selection target and applying the first calculation value as a corrected counter value, and an operation of repeating the selection target extracting operation and the first extraction counter correcting operation.

The first extraction counters may correspond to clocks, and time change speeds of the clocks for the selection targets are applied differently according to the first selection ratio.

The method may further include an operation of, when the plurality of selection targets are extracted at the same time point, determining the sequence of extractions at random.

The method may further include an operation of, when a least common multiple included in the terms of the first selection ratio is present, determining a correction value added to terms corresponding to the selection targets and calculating a final ratio added to the terms, wherein the correction value is a value that prohibits the terms in the final ratio have a least common multiple, and the first extraction counter speed determining operation may include an operation of determining the speeds of the first extraction counters for the selection targets based on the final ratio.

The correction value may be a value between 0 and 1, which is determined for the selection targets by a random function.

The selection target extracting operation and the repetition operation may include an operation of extracting a specific selection target by increasing the counter value at a selection target extracting timing.

The method may further include, when a plurality of detailed selection items are included in a selection target, a second selection ratio receiving operation of receiving a second selection ratio for the plurality of detailed selection items in the selection target, by the computer, a second extraction counter speed determining operation of determining speeds of second extraction counters for the detailed selection items, wherein the second extraction counters are set for the detailed selection items and wherein the speeds are proportional to the second selection ratio, a detailed selection item extracting operation of, if a second extraction counter for a specific detailed selection item reaches the reference value as the counter value is increased at a speed determined for the second extraction counter, extracting a detailed selection item, the counter value of which reaches the reference value, and a second extraction counter initializing operation of initializing a counter of the extracted detailed selection item.

The method may further include an operation of, when the selection target is a training type, calculating a first selection ratio for a plurality of training types.

The first selection ratio calculating operation may include an operation of acquiring current state data for a specific body portion of a specific user, an operation of acquiring training level data by applying the current state data and normal state data to a training level calculation model, an operation of calculating evaluation data for a specific training type by applying the acquired training level data to a training ratio determination model, and an operation of determining the first selection ratio by calculating a ratio of evaluation data for a plurality of training types, the current state data may be data for performance of a specific training type for a specific body portion of the user, the normal state data may be data for performance of a specific training type for a specific body portion of a normal person, and the training level data may be value data that represents a level by which performance of training according to the current state for a specific body portion of the user is allowed.

In accordance with another embodiment of the inventive concept, there is provided a program for determining a sequence of extractions by using a computer, which is coupled to a computer that is a piece of hardware and is stored in a medium to execute the method.

BRIEF DESCRIPTION OF THE FIGURES

The above and other objects and features will become apparent from the following description with reference to the following figures, wherein like reference numerals refer to like parts throughout the various figures unless otherwise specified, and wherein:

FIG. 1 is a flowchart of a method for determining a sequence of extractions by using a computer according to an embodiment of the inventive concept;

FIG. 2 is a flowchart of a method for determining a sequence of extractions, by which a selection target is extracted based on whether a counter value of a first extraction counter reaches a reference value according to an embodiment of the inventive concept;

FIG. 3 is an exemplary view illustrating a change of first extraction counters of a plurality of selection targets when a selection target is extracted based on whether a counter value reaches a reference value according to an embodiment of the inventive concept;

FIG. 4 is a flowchart of a method for determining a sequence of extractions, by which a selection target is extracted based on whether a counter value of a first extraction counter is maximal according to an embodiment of the inventive concept;

FIG. 5 is an exemplary view illustrating a change of first extraction counters of a plurality of selection targets when a selection target is extracted based on whether a counter value corresponds to a maximum value according to an embodiment of the inventive concept;

FIG. 6 is a flowchart of a method for determining a sequence of extractions by using a computer, the method further including a process of setting the sequence when a plurality of selection targets are extracted at the same time point according to an embodiment of the inventive concept;

FIG. 7 is a flowchart of a method for determining a sequence of extractions by using a computer, the method further including a process of generating a final ratio while reflecting a correction value according to an embodiment of the inventive concept;

FIG. 8 is a flowchart of a method for determining a sequence of extractions by using a computer, the method further including a process of extracting a plurality of detailed selection items in a selection target according to an embodiment of the inventive concept;

FIG. 9 is an exemplary view of a relationship between a selection target and a detailed selection item according to an embodiment of the inventive concept;

FIG. 10 is a flowchart of a method for determining a sequence of extractions by using a computer, the method further including a process of extracting a plurality of detailed selection items in a selection target according to another embodiment of the inventive concept;

FIG. 11 is a flowchart of a method for determining a sequence of extractions by using a computer, the method further including a process of generating a first selection ratio according to an embodiment of the inventive concept;

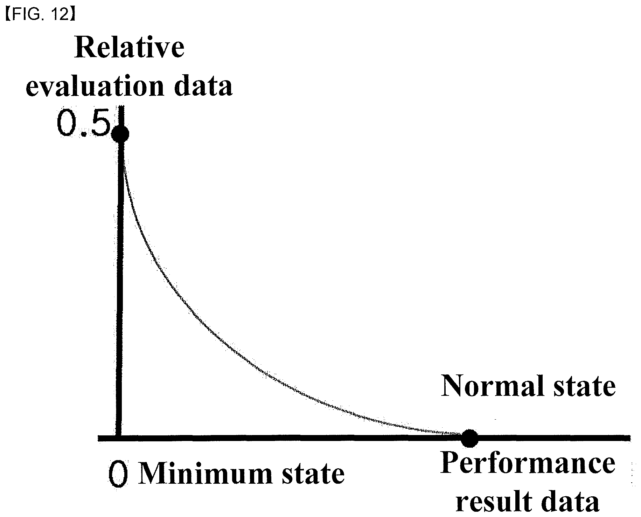

FIG. 12 is an exemplary graph by a calculation equation in a current state evaluation model according to an embodiment of the inventive concept; and

FIG. 13 is an exemplary graph according to a training ratio calculation model according to an embodiment of the inventive concept.

DETAILED DESCRIPTION

In the specification, a computer includes all various devices that may provide a user with a result by performing a calculation. For example, the computer may correspond to a smartphone, a tablet Pc, a cellular phone, a personal communication service (PCS) phone, a mobile terminal of a synchronous/asynchronous international mobile telecommunication (IMT)-2000, a palm personal computer (PC), and a personal digital assistant (PDA), in addition to a desktop computer and a notebook. Further, the computer may be a server that receives a request from the client and process information.

In the specification, 'selection targets' refer to targets that are to be provided in sequence. For example, the 'selection targets' refer to a plurality of training types that have to be performed by a rehabilitation training patient. That is, because a rehabilitation patient may perform only one rehabilitation training type at a specific time point, the 'selection targets' refer to a plurality of training types that have to be suggested to the patient in a suitable sequence. The 'selection targets' are not limited to the suggested rehabilitation training types, but may include various targets, of which sequence has to be set while the plurality of targets have a specific ratio.

In the specification, 'detailed selection items' refer to a plurality of detail items included in the category of the 'selection targets'. For example, if the 'selection targets' are rehabilitation training types, the targets of which are specific body portions, the 'detailed selection items' may be a plurality of game contents, through which the specific body portions may be trained.

In the specification, a 'first selection ratio' refers to a ratio of the numbers of extractions of the selection targets that are extracted in a specific sequence. That is, when the selection targets are extracted n times, the 'first selection ratio' refers to a value which the ratios of the numbers of extractions of the plurality of selection targets included in the n times have to be the same as or similar to.

In the specification, a 'second selection ratio' refers to a ratio which the plurality of 'detailed selection items' have to be the same as or similar to within the number of extractions of a specific selection target. For example, when the specific selection targets include detailed selection item A, detailed selection item B, and detailed selection C and the second selection ratio is 3:2:1, detailed selection item A is extracted six times, detailed selection item B is extracted four times, and detailed selection item C is extracted two times if the detailed selection items are extracted twelve times.

Hereinafter, a method for determining a sequence of extractions by using a computer and a computer program according to embodiments of the inventive concept will be described with reference to the drawings.

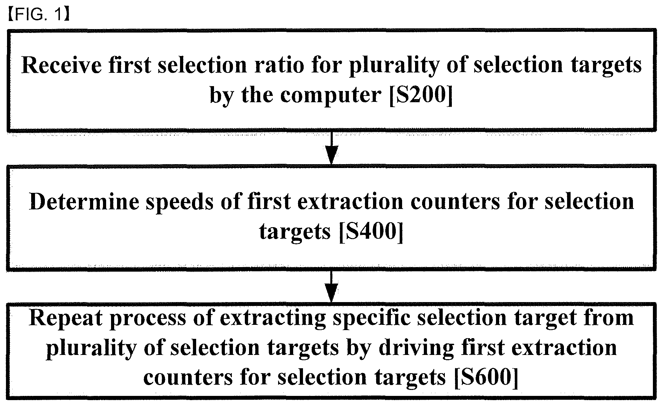

FIG. 1 is a flowchart of a method for determining a sequence of extractions by using a computer according to an embodiment of the inventive concept.

Referring to FIG. 1, the method for determining a sequence of extractions by using a computer according to an embodiment of the inventive concept includes an operation of receiving a first selection ratio for a plurality of selection targets by the computer (S200) (a first selection ratio receiving operation), an operation of determining speeds of first extraction counters for the selection targets (S400) (a first extraction counter speed determining operation), and an operation of repeating a process of extracting a specific selection target from the plurality of selection targets by driving the first extraction counters for the selection targets (S600). Hereinafter, the operations will be described in detail.

The computer receives a first selection ratio for a plurality of selection targets (S200) (a first selection ratio receiving operation). That is, the computer receives a ratio at which the plurality of selection targets have to be selected. In an embodiment, when rehabilitation training for a plurality of body portions is performed, the numbers or frequencies of the rehabilitation training which has to be performed are different according to the states of the body portions. Accordingly, the computer sets the first selection ratio by calculating a rehabilitation training performance ratio for the body portions, or receive the rehabilitation training performance ratio for the body portions from an external device (for example, a terminal device of a medical staff or a rehabilitation therapist) as the first selection ratio. The computer determines a speed of first extraction counters for the selection targets (S400) (a first extraction counter speed determining operation). The first extraction counters are set for the respective selection targets, and the speeds of the first extraction counters are determined in proportion to the first selection ratio. That is, the first extraction counters function to determine times at which the selection targets are extracted. That is, if a counter value reaches a reference value that is a reference extraction condition as a first extraction counter corresponding to a specific selection target increases the counter value, the computer extracts the corresponding selection target as a performance target of the corresponding performance timing.

The computer gives reference arrival speed of the first extraction counters given to the selection targets according to the numerical values of the selection targets on the first selection ratio. For example, when a first selection ratio (for example, a performance ratio) for a first rehabilitation training type (for example, a wrist rotation rehabilitation exercise), a second rehabilitation training type (for example, a motion rehabilitation exercise of bending a finger), and a third rehabilitation training type (for example, a motion rehabilitation exercise of bending a wrist) is received as 3:2:1, the computer sets a ratio of the reference arrival speed of the first rehabilitation training type to that of the third rehabilitation training type to three times or more and sets a ratio of the reference arrival speed of the second rehabilitation training type to that of the third rehabilitation training type to two times or more. As an embodiment, the first extraction counters correspond to clocks. The time change speeds of the clocks for the selection targets are applied differently according to the first selection ratio. The reference value for extracting a selection target becomes a specific time point of the clock. For example, the computer sets 12 hour or 24 hour as the reference value, and different time change speeds are applied to the given clock for the respective selection targets.

When the clock is applied as the first extraction counter and the reference extraction value of the selected target is set to 12 o'clock or 24 o'clock, the first extraction counter is automatically initialized after the selection target is extracted. The clock is automatically initialized as the time becomes 0 o'clock if the time passes 24 o'clock (for example, in an electronic clock that does not distinguish a.m./p.m.) or 12 o'clock (for example, in a clock, a second hand and a minute hand of which rotate). Accordingly, the computer does not perform a separate initialization process, and automatically initializes the first extraction counter after the selection target is extracted as the selection target reaches the reference value.

Thereafter, the computer repeats a process of extracting a specific selection target from the plurality of selection targets by driving the first extraction counters for the respective selection targets (S600). That is, the computer repeats the selection target extracting process for the respective timings at which the sequence of the selection targets is generated or respective selection target extraction timings.

As an embodiment, the computer generates the sequence of the selection targets by repeating extractions of the selection targets at once at a timing at which the sequence of the selection targets is generated. Further, as another embodiment, the computer performs the selection target extracting process one time whenever extracting the selection targets.

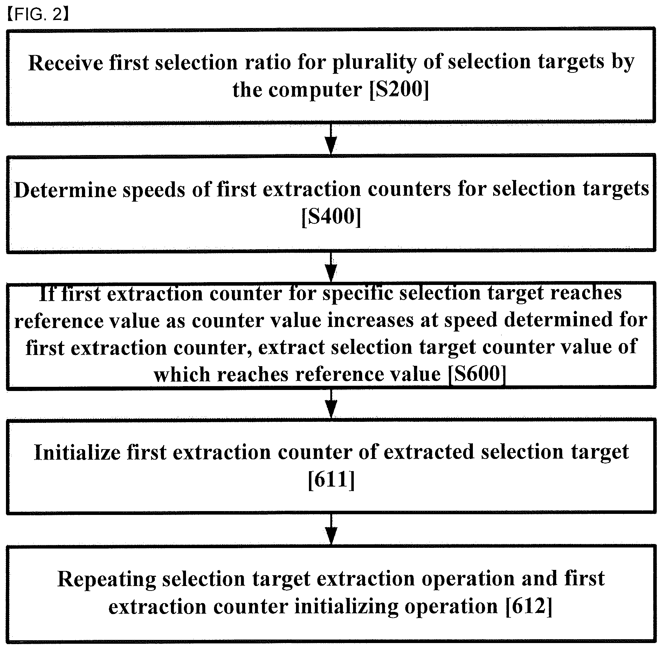

Various schemes may be applied as the scheme of performing the selection target extracting process. As in FIG. 2, an embodiment of the operation of repeating the selection target extracting process includes a selection target extracting operation (S610) of, if a first extraction counter for a specific selection target reaches the reference value as the counter value increases at a speed determined for the first extraction counter, extracting the selection target the counter value of which reaches the reference value (S610), a first extraction counter initializing operation of initializing the first extraction counter of the extracted selection target (S611), and repeating the selection target extracting operation and the first extraction counter initializing operation (S612).

If a first extraction counter for a specific selection target reaches a reference extraction value as the computer increases the counter value at a speed determined for the first extraction counter, a selection target the counter value of which reaches the reference value is extracted (S610) (a selection target extracting operation). For example, the computer drives the first extraction counters corresponding to the selection targets at a selection target extraction timing. If a specific first extraction counter reaches the reference extraction value as the counter value for the first extraction counter increases according to a given speed, a selection target corresponding to the first extraction counter is extracted. Thereafter, the computer initializes the first extraction counter for the extracted selection target (S611) (a first extraction counter initializing operation). That is, the computer initializes the first extraction counter corresponding to the extracted selection target, and the other first extraction counters are maintained at the increased counter values. Thereafter, the computer repeats the selection target extracting operation and the first extraction counter initializing operation (S612). That is, the computer increases the counter value of the initialized first extraction counter at a specific speed from the initial state, and increases the counter values of the other first extraction counters at a given speed from the maintained counter values. Then, if a specific first extraction counter reaches the first value, the computer extracts a selection target corresponding to the first extraction counter and initializes the first extraction counter. The computer repeats the process at a timing at which it is necessary to extract the selection target.

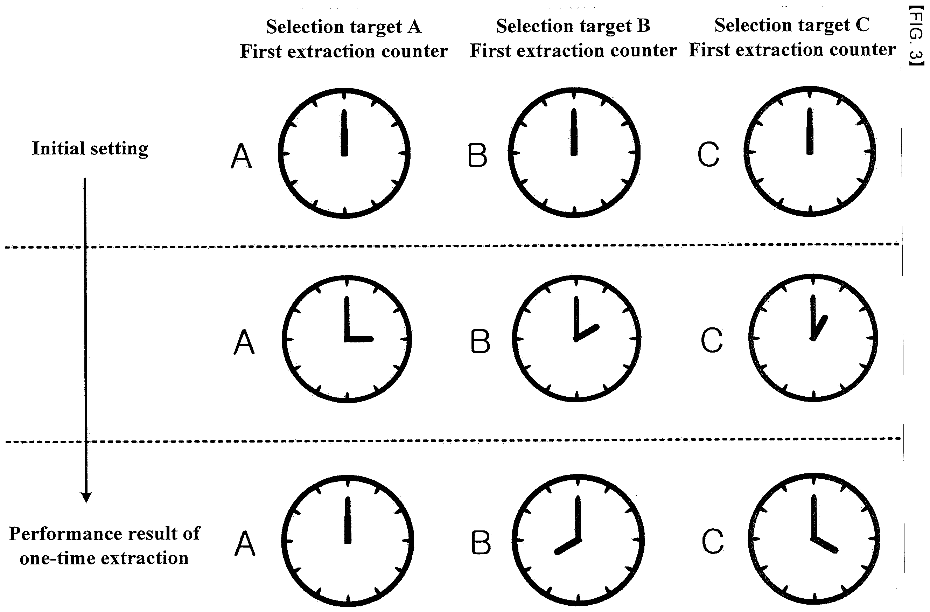

In detail, as in FIG. 3, when the computer has a selection ratio of selection target A:selection target B:selection target C of 3:2:1, the counter value of the first extraction counter of selection target A increases at a speed of 3 per unit time, the counter value of the first extraction counter of selection target B increases at a speed of 2 per unit time, and the counter value of the first extraction counter of selection target C increases at a speed of 1 per unit time. As the first extraction counters of the selection targets increases at a selection target extraction timing, the first extraction counter of selection target A reached the reference value, and the computer initializes the first extraction counter of selection target A while extracting selection target A. Then, the ratio of the increase speeds of the counter values is 3:2:1, the first extraction counter of selection target B reaches 8 o'clock and the first extraction counter of selection target C reaches 4 o'clock, and the first extraction counters of selection target B and selection target C maintain the corresponding values before the next extraction timing. At a timing at which the next selection target is extracted, the computer increases the counter value from 0 for the first extraction counter of selection target A, and increases the counter values according to the given increase speeds, from a value corresponding to 8 o'clock for the first extraction counter of selection target B and from a value corresponding to 4 o'clock for the first extraction counter of selection target C. Through this, as the first extraction counter of selection target B reaches the reference extraction value and selection target B is extracted, the first extraction counter of selection target B is initialized, and the first extraction counter of selection target A and the first extraction counter of selection target C reach 6 o'clock and are stopped. Through the process, if the selection target extracting process is repeated six times, selection target A is extracted three times, selection target B is extracted two times, and selection target C is extracted once so that the selection targets are extracted in conformity with the first selection ratio. Accordingly, selection target A, selection target B, and selection target C are provided to times (or densities) that are in conformity with the first selection ratio while their orders are mixed (that is, they are provided alternately).

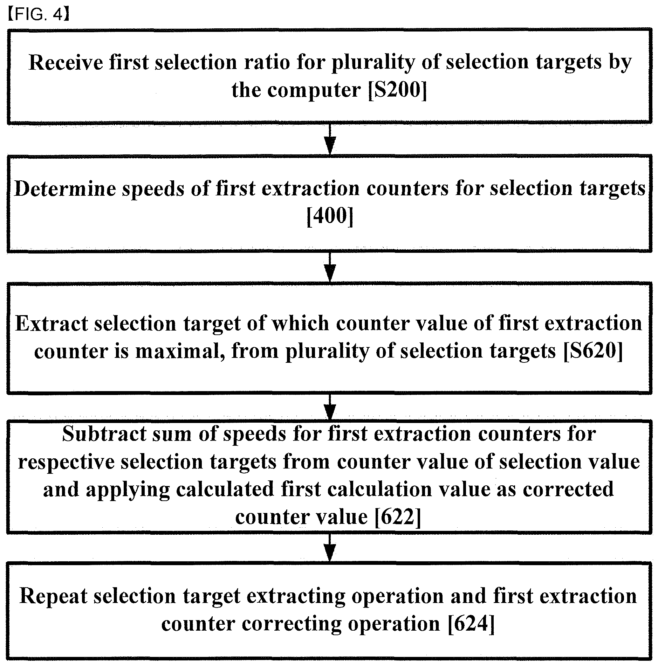

Further, as in FIG. 4, another embodiment of the operation of repeating the selection target extracting process includes a selection target extracting operation of extracting a selection target of which the counter value of the first extraction counter is maximal, from a plurality of selection targets as the counter values of the first extraction counters are increased at a predetermined speed (S620), a first extraction counter correcting operation of calculating a first calculation value by subtracting a sum of the speeds for the first extraction counters for the respective selection targets from the counter value of the selection value and applying the calculated first calculation value as a corrected counter value (S622), and repeating the selection target extracting operation and the first extraction counter correcting operation (S624).

As the computer increases the counter values at speeds determined for the respective first extraction counters, a selection target, of which the counter value of the first extraction counter is maximal, is extracted from the plurality of selection targets (S620) (a selection target extracting operation). That is, the computer allows the first extraction counters to increase the counter values for a unit time, and as a result of the increases of the counter values, extracts a selection target, of which the counter value of the first extraction counter is maximal, from the plurality of selection targets.

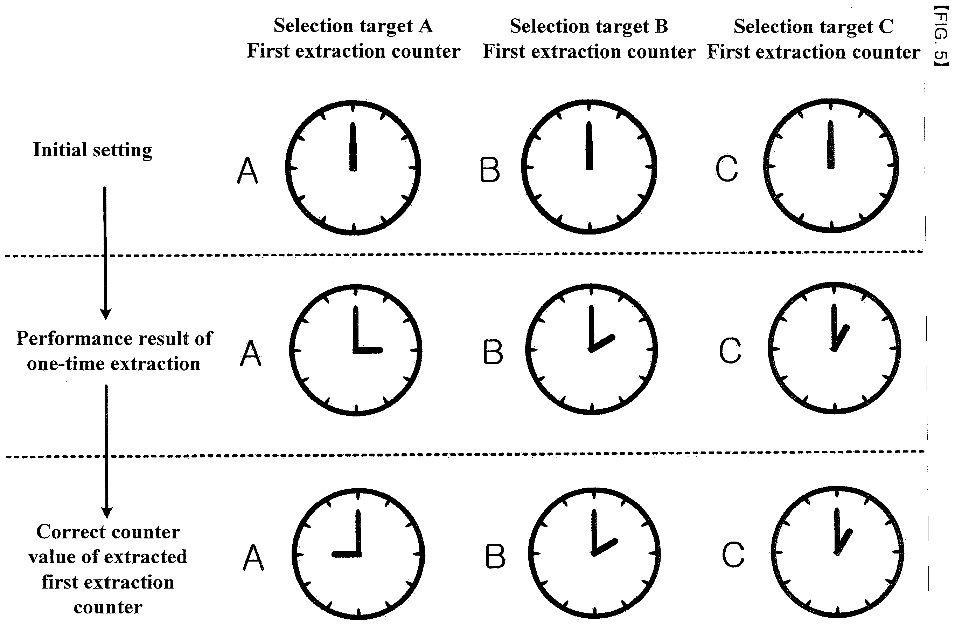

Thereafter, the computer calculates a first calculation value by subtracting a sum of the speeds for the first extraction counters for the selection targets from the counter value of the selection target and applies the first calculation value as a corrected counter value (S622) (a first extraction counter correcting operation). That is, as in FIG. 5, the computer performs a process of extracting a selection target corresponding to a first extraction counter, of which a specific counter value is maximal, and then correcting the counter value of the corresponding selection target such that the counter value of the corresponding selection target is not maximal. The computer calculates a first calculation value by subtracting a value of the sum of the speeds of the first extraction counters, which is changed for a unit time, from the counter value of the extracted selection target. To achieve this, an embodiment of the first extraction counter may have a positive number or a negative number as a counter value.

Thereafter, the computer repeats the selection target extracting operation (S620) and the first extraction counter correcting operation (S622) (S624).

Further, as another embodiment, the selection target extracting operation S620, a process of, when all the selection target extraction counters are zero, determining that a maximum value is not present and increasing the counter value of the extraction counter is performed once more. If the states of all the first extraction counters become an initial state (that is, 0) after the selection target extraction operation and the first extraction counter correcting operation are repeated in their initial states, one cycle is completed. The computer makes the ratio of the speeds of the first extraction counters for the selection targets and the ratio of the numbers of extractions of the selection targets in one cycle in the same way by prohibiting a selection target from being extracted when all of the first extraction counters reach the initial state.

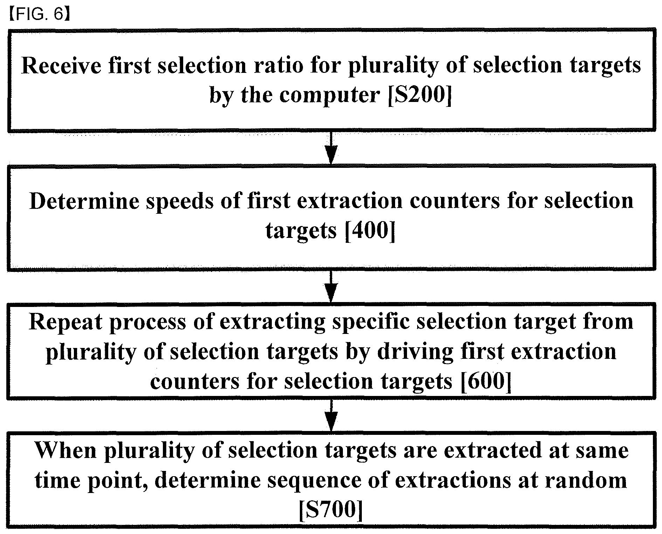

Further, as in FIG. 6, another embodiment may further include an operation of determining a sequence of extractions at random when a plurality of selection targets are extracted at the same time point (S700). When the values of the terms of the selection ratio have a least common multiple, the plurality of selection targets may reach a reference value at the same time point. For example, the first extraction counters are initialized while the selection targets are extracted when the reference value is reached as operations S610 to S612 are performed in a state in which selection target A, selection target B, and selection target C have a selection ratio of 3:2:1, selection target A, selection target B, and selection target C reach the reference value at the same time in the fourth extraction. Accordingly, when two or more selection targets are extracted at the same time, the computer sets the sequence of the plurality of selection targets at random.

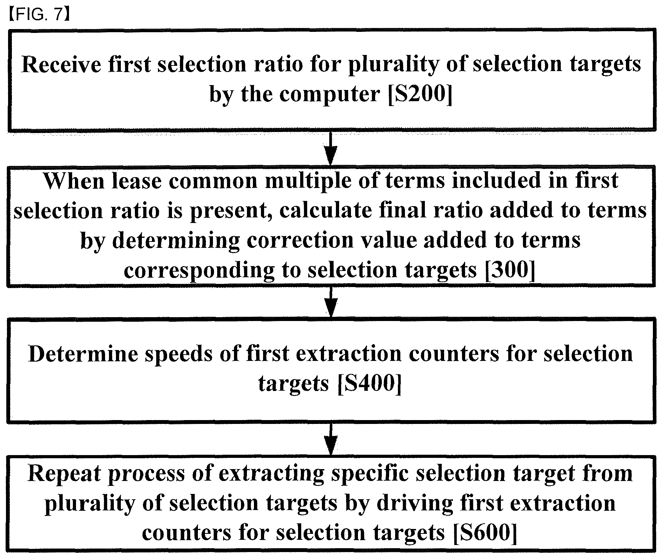

Further, as in FIG. 7, another embodiment further includes an operation of, when the terms of the first selection ratio have a least minimum multiple, determining a correction value added to the terms corresponding to the selection targets and calculating a final ratio added to the terms (S300) (a final ratio calculating operation). The correction value is a value for prohibiting the terms of the final ratio from having a lease minimum multiple. As an embodiment, the correction value is a value between 0 and 1, which is determined for the selection targets by a random function. The sequence until the reference value is reached or the sequence until a maximum value of the first extraction counters is reached should not be changed as the correction value is added. Accordingly, the computer extracts a value between 0 and 1, which is small, as a correction value.

Further, when a final ratio is calculated by adding the correction value to eliminate a least common multiple between the numerical values corresponding to the selection targets in the first selection ratio, in the operation (S400) of determining the speeds of the first extraction counters, the speeds of the first extraction counters for the selection targets are determined based on the final ratio. That is, the computer determines a counter value increase speed based on the numerical values (that is, the values of the terms) corresponding to the selection targets in the final ratio.

Further, in the selection target extracting operation (S610 or S620) and the repetition operation (s612 or S624), selection targets are extracted in various schemes. In an embodiment, the computer extracts a specific selection target by increasing the counter value at a selection target extracting timing. Further, the computer extracts the next selection target if the timing at which the next selection target has to be determined advents. For example, when a plurality of rehabilitation training types (for example, rehabilitation training type A, rehabilitation training type B, and rehabilitation training type C) have to be performed, rehabilitation training type A is extracted and is performed by the user, and if performance of the next rehabilitation training is requested by the user, a rehabilitation training type corresponding to the next order is extracted.

Further, in another embodiment, the computer generates a sequence by performing extractions a predetermined number of times. For example, if extractions have to be performed on a plurality of rehabilitation training types (for example, rehabilitation training A, rehabilitation training B, and rehabilitation training C), the computer generates a sequence of performance of rehabilitation training by repeatedly extracting the selection targets at a timing at which the sequence of the plurality of selection targets is generated.

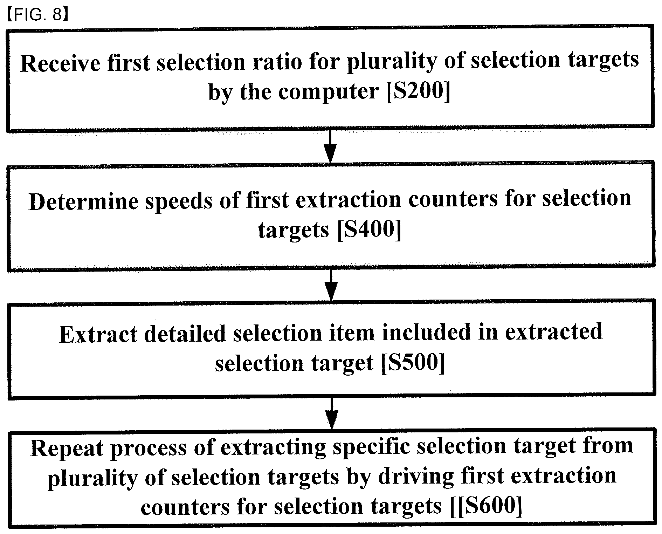

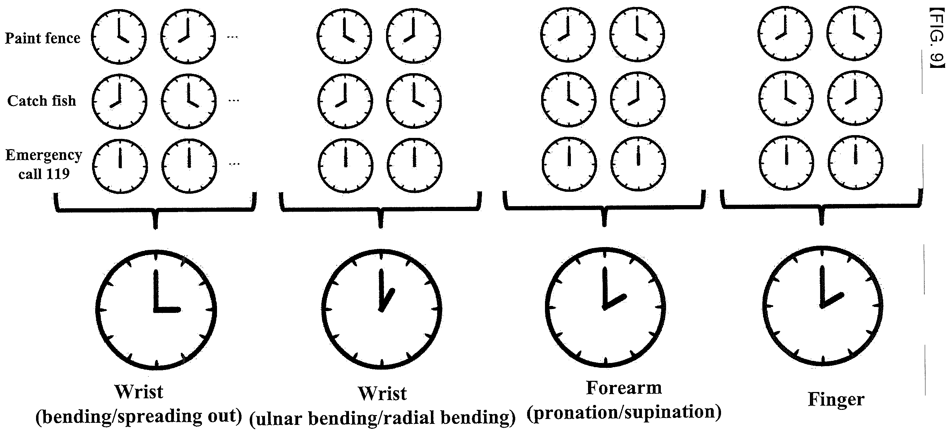

Further, as illustrated in FIG. 8, another embodiment further includes an operation (S500) of extracting a detailed selection item included in the extracted selection target. The selection target corresponds to a specific high level classification, and includes a plurality of detailed selection items. For example, as in FIG. 9, when the selection target is a rehabilitation training type of a specific body portion, a rehabilitation training game included in the rehabilitation training type corresponds to the detailed selection item. That is, when the selection target is a rehabilitation training type for training of folding and unfolding a finger, a butterfly catching game, an orange squeezing game, and the like may correspond to the detailed selection time. If a specific selection target is extracted through a first extraction counter, to which a first selection ratio is applied, the computer extracts a specific one from the plurality of detailed selection items included in the selection target.

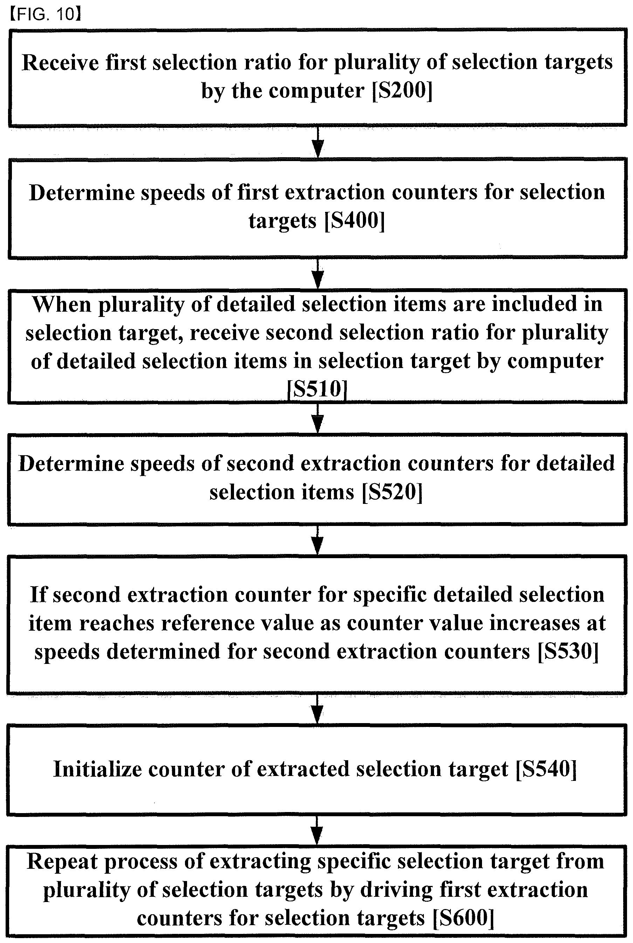

In detail, as in FIG. 10, an embodiment of the operation (S500) of extracting the detailed selection item further includes, when a plurality of detailed selection items are included in a selection target, a second selection ratio receiving operation of receiving a second selection ratio for the plurality of detailed selection items in the selection target, by the computer (S510), a second extraction counter speed determining operation of determining speeds of second extraction counters for the detailed selection items, wherein the second extraction counters are set for the detailed selection items and wherein the speeds are proportional to the second selection ratio (S520), a detailed selection item extracting operation of, if a second extraction counter for a specific detailed selection item reaches the reference value as the counter value is increased at a speed determined for the second extraction counter, extracting a detailed selection item, the counter value of which reaches the reference value (S530), and a second extraction counter initializing operation of initializing the counter of the extracted selection target (S540). The computer receives a second selection ratio for selecting a specific one from the plurality of detailed selection items included in a specific selection target (S510). For example, because a difference of rehabilitation effects and a difference of tastes of users (that is, patients) are present according to a game for rehabilitation training that is a detailed selection item, the computer receives a second selection ratio to provide the detailed selection item at a sequence or ratio that is suitable for the user. Thereafter, the computer determines a counter value increase speed of a second extraction counter by applying the second selection ratio (S520). If a second extraction counter for a specific detail selection item reaches the reference value as the counter value is increased at a speed determined for the second extraction counter, the computer extracts a detail selection item, the counter value of which reaches the reference value (S530) (a detailed selection item extracting operation). Thereafter, the computer initializes the second extraction counter of the extracted detailed selection item (S540) (a second extraction counter initializing operation), and the second extraction counter of another detailed selection item maintains the counter value. Thereafter, the computer repeats a process of selecting a detailed selection item based on the second extraction counter in the extracted specific selection target while repeatedly extracting the selection targets based on the first extraction counters.

As an embodiment, the second extraction counters for the detailed selection items in the specific selection target are stopped when the counter value of the first extraction counter changes for extraction of the selection target, and the counter values of the second extraction counters change after the selection target is determined by the first extraction counter. Through this, the detailed selection items for the selection targets are independently extracted by adjusting only the second extraction counters of the detailed selection item included in the extracted selection target.

Further, in an embodiment of the first selection ratio receiving operation (S200), a first selection ratio for a plurality of training types is calculated when the selection target is a training type. The training is an act that is performed to improve or enhance the function of a specific body portion of the user. That is, when the function of a body portion of a specific user does not reach a state of a normal person (that is, when the user is a patient that requires rehabilitation for a specific body portion), the training is rehabilitation training that is performed to improve a specific body portion. Further, the specific user corresponds to a normal person, and when the function of a body portion of the user is to be improved further, the function improving exercise (for example, a muscular exercise or a muscle endurance exercise) performed on a specific body portion corresponds to the training. Further, the training type is a type that has to be performed as training of a specific body portion. The training type may mean a type of a motion that may be performed by a specific body portion. For example, when the body portion is a 'hand', the 'training type' includes folding and unfolding a finger, bending and stretching of a wrist, and rotation of a wrist. Further, the 'training type' may mean a detailed type of a task (for example, orange squeezing game contents and butterfly catching game contents for 'folding and unfolding a finger') that moves a specific body portion.

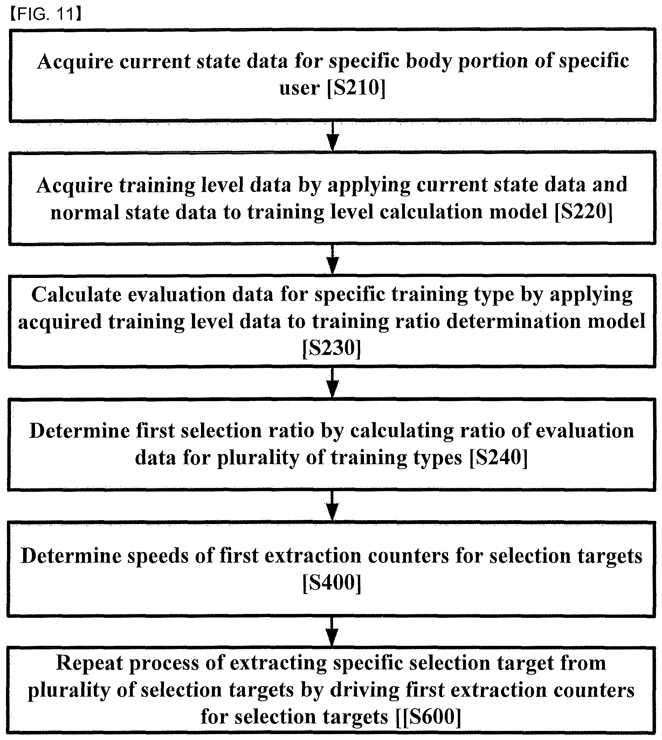

As in FIG. 11, a detailed embodiment of calculating the first selection ratio includes an operation of acquiring current state data for a specific body portion of a specific user (S210), an operation of acquiring training level data by applying the current state data and normal state data to a training level calculation model (S220), an operation of calculating evaluation data for a specific training type by applying the acquired training level data to a training ratio determination model (S130), and an operation of determining the first selection ratio by calculating a ratio of evaluation data for a plurality of training types (S240).

The computer acquires current state data for a specific body portion of a specific user (S210). The current state data is data for performance of a specific training type for a specific body portion of the user. That is, the computer acquires data for determining a current state for a body portion of the user (for example, a patient).

As an embodiment, when a range of motion (ROM) of a joint is measured, current state data is acquired by measuring a current specific motion of the joint of the user. The range of motion of the joint may be measured through a rehabilitation training device (for example, a body state measuring device or a glove type/hand-mounted rehabilitation device) that performs training while being mounted on a specific body portion of the user. For example, when the body portion is a hand, the training type includes rotation of a wrist, bending and stretching of a wrist, rotation of a forearm, folding and unfolding of a finger, and the computer acquires measurement data of a range of motion of a joint for the training type as current state data as the user perform a motion while wearing a hand-mounted measurement device. Further, for example, when a range of motion of a joint of a shoulder is measured, the range of motion of the joint is measured by using a measurement sensor device attached to the shoulder portion or a device disposed on the bottom surface to provide a 2-dimensional motion of the shoulder.

The computer acquires state evaluation data by applying the current state data and the normal state data to the current state evaluation model (S220). First, the computer acquires normal state data for a specific training type of a specific body portion. The normal state data is data for performance of a specific training type for a specific body portion of a normal person, and

The computer may acquire normal state data in various schemes. As an embodiment, a manager designates normal state data of a specific training type to the computer. In detail, when the computer is a terminal device carried by the user, it receives and stores optimum normal state data as it performs wireless communication with an external management server. Further, in another embodiment, the normal state data is determined by a maximum point on a graph of the number of normal persons to the performance result data of a specific training type. In detail, when the computer is a management server, it may accumulate a performance result for a specific training type of normal persons, and generates a graph of the number of normal persons to the corresponding performance result data. The performance result data value on the graph, which corresponds to the largest number of persons may be determined as normal state data. Further, in another embodiment, the normal state data is determined by an average value of the performance result data acquired by a plurality of normal persons.

The state evaluation data is data that is evaluated by comparing the current state of the user with a state of a normal person to determine a level of training that is in conformity with the current state of the user. It may not be proper to directly apply current state data measured by a body state measurement device or a rehabilitation device or current state data that is a specific test result performed on the user by a medical staff. To achieve this, the computer converts the current state data to data that is suitable for calculating a training ratio. That is, the computer performs a process of evaluating which state the current state is, by comparing the current state with the state of a normal person.

In an embodiment of the current state evaluation model, the state evaluation data is calculated by calculating a third numerical value that is in conformity with the state of the user through a specific calculation equation in a numerical range between a first numerical value corresponding to a minimum state and a second numerical value corresponding to a normal person. That is, the calculation equation may be an equation for an increasing function or a decreasing function that changes between a first numerical value corresponding to a minimum state (that is, a state that does not perform a training type at all) and a second numerical value corresponding to a state of a normal person, and the computer may apply a third numerical value that is in conformity with data on a specific current state between the minimum state and the state of a normal person.

The calculation equation included in the current state evaluation model may be a functional equation that increases or decreases in direct proportion, and may be an equation that is in conformity with a specific function other than a linear function. For example, in a motion of rotating a wrist, a motion of a small rotation angle may be determined to be a high state improvement in an initial rotation zone in a specific direction, but in a zone that is close to a maximum rotation range, an improvement of the performance result by the same rotation angle may be determined to be a low state improvement as compared with an initial rotation zone. Accordingly, when the state of a patient is evaluated, the same difference value of the current state data measured previously and the current state data measured currently may be determined differently according to the previous state (that is, the change rate of the state evaluation data may be different).

For example, an example of calculating state evaluation data based on the following equation will be described in detail. Meanwhile, the equation of the current state evaluation model according to the inventive concept is not limited at all.

$$Z_{i=\frac{1}{2\bar{n}^{1.2}}(\bar{n}-x_i)^{1.2}}$$

($\bar{n}$: normal state data, $x_i$: current stat data, and $z_i$: state evaluation data)

The numerical range of the equation is set from 0 to 0.5, and becomes closer to a maximum value of 0.5 as the state is worse (that is, the state becomes closer to a minimum state) and becomes closer to 0 as the state becomes better (that is, $x_i$ becomes closer to n). Further, because the equation has a form of an exponential function or a functional form (that is, a graph form before a minimum point that is downwardly convex) in which functional values decrease as the change rate increases, a difference of the state evaluation data values according to the same difference value of the current state data in an area in which the state becomes better (that is, an area that is close to that of a normal person) is smaller than a difference of state evaluation data according to a difference value (for example, an increase of a motion range of rotation of a wrist by a specific angle) of specific current state data in an area in which the state is not good (that is, an area that is close to that of the minimum state).

An embodiment of the calculation equation in a current state evaluation model in which a functional value decreases while a change rate increases as current state data increases may be an equation in which a difference value between the normal state data and the current state data is raised to the R-th power (R is a real number that is greater than 1).

Further, as an embodiment, the computer applies an equation that is in conformity with features of a specific body portion or a training type as the calculation equation included in the current state evaluation model. Different equation forms may be applied while reflecting the features according to the body portion or the training type, and only a constant value included in the same equation form may be adjusted.

Further, in another embodiment, when a specific training type has a motion that is symmetrical to the reference posture, the state evaluation data on the specific training type is calculated by averaging first state evaluation data corresponding to a motion in a first direction and second state evaluation data corresponding to a motion in a second direction. For example, because a wrist may be rotated in opposite directions from a reference state (that is, a state in which a joint is not rotated), the current state on the respective directions (that is, the first direction and the second direction) have to be evaluated individually. Further, for example, when the training type is bending of a wrist, the bending of the wrist in the upward and downward directions has to be evaluated individually. That is, the computer acquires state evaluation data (that is, first state evaluation data and second state evaluation data) in the respective directions (that is, the first direction and the second direction) for the body portion having a symmetric motion. Thereafter, the computer acquires final state evaluation data on a specific training type of the corresponding body portion as the first state evaluation data and the second state evaluation data are averaged.

Further, in another embodiment, when the training type has a symmetrical motion of a specific body portion, the current state evaluation model includes an equation of calculating a numerical value that reflects the states in the respective directions as state evaluation data. For example, when a first state in which a wrist is inclined by 30 degrees in the first direction from the reference location and is not rotated at all in the second direction and a second state in which the wrist is inclined by 15 degrees in the first direction and is inclined by 15 degrees in the second direction in a training type of rotation of the wrist, the final state evaluation data on the first state and the final state evaluation data on the second state may be calculated in the same way by applying an equation (that is, an equation in which the state evaluation data increases in direct proportion to the normal state data) of a function that is in direct proportion to the normal state data and the current state data. Because it is necessary for the first state and the second state to be evaluated to be different when the first state and the second state are displayed on the numerical values of the final state evaluation data when the state of the patient is evaluated during rehabilitation training, the computer uses an equation in which a deviation from a final state evaluation value in a specific direction.

In an embodiment of the current state evaluation model for this, a difference value between the normal state data and the current state data is raised to the R-th power (R is a real number that is greater than 1). For example, as in FIG. 3, in an equation having a functional form in which a functional value decreases while a change rate increases as current state data increases, because an absolute value of the change rate for the state evaluation data is larger as the current state data is small (that is, the state evaluation data rapidly changes as the current state data is small), the deviation of the motion in a specific direction may be reflected on the numerical values.

The computer calculates training level data on a specific training type by applying the acquired state evaluation data to a training ratio determination model (S230). The training level data means a training level of the user that is suitable for state evaluation data on a specific training type of a specific body portion. The training ratio of a plurality of training types has to be differently set according to the state of the patient. For example, the training type of a patient that results in a performance result that is close to that a normal person may be provided less, and the training type that is determined to be a state in which a patient may be improved with a high possibility may increase the training effect by increasing the training ratio.

In an embodiment of the training ratio determination model, training level data in the minimum state and the state of a normal person is set to 0 and an equation has a specific training level data value in a specific state between the minimum state and the normal state. When a specific training type is performed, in a minimum state (for example, a state in which a specific body portion cannot be moved at all according to a specific training type), provision of the corresponding training type to the user cannot be helpful and may deteriorate an interest in the rehabilitation training of the user. Accordingly, the computer sets the training level data in the minimum state to 0. Further, when state evaluation data on a training type of a specific body portion is calculated to be a level of a normal person, the corresponding user does not require rehabilitation training for the training type of the corresponding body portion, and accordingly, the computer sets the training level data of the corresponding training type to 0. Further, an equation is determined such that a specific continuous function is provided between a minimum state and a state of a normal person. An embodiment of the continuous function may be a function of determining training level data based on a state evaluation data value in a specific numerical range (that is, a range that is greater than 0 and smaller than 1). The equation included in the training ratio calculation model may be set differently according to the body portion or the training type.

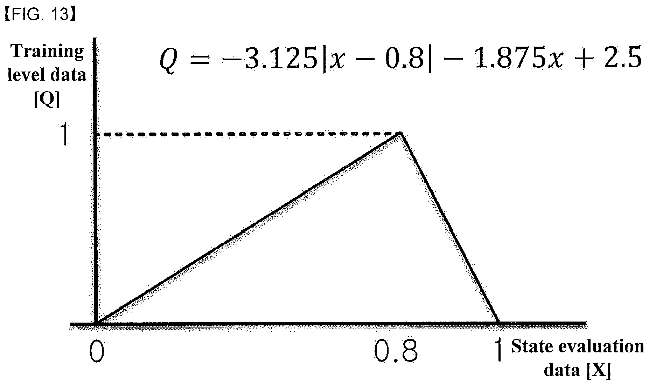

For example, as in FIG. 13, the equation may be included in the training ratio determination model such that a maximum training ratio (that is, a maximum value) is obtained at a specific state evaluation data value. The state evaluation data value having a maximum value may be set by a medical staff or may be set and adjusted by analyzing levels of rehabilitation (that is, state improvement states) of the users by the computer.

The computer determines a first selection ratio between a plurality of training types based on training level data on the plurality of training types (S240). In an embodiment of a scheme of calculating the first selection ratio, the computer calculates a ratio by directly comparing training level data of the training types. For example, when the training level data of training type A, training type B, and training type C is 0.6, 0.5, and 0.4, respectively, the computer determines 0.6:0.5:0.4 that is a ratio between the training level data for the respective training types as a performance ratio (that is, the first selection ratio). Further, the computer may calculate a final ratio that is a ratio of integers, which is obtained by multiplying the performance ratio by a specific natural number.

The method of determining a sequence of extractions by using a computer according to an embodiment of the inventive concept may be coupled to a computer that is a piece of hardware to be implemented by a program (or an application) and stored in a medium.

The program may include a code that is coded in a computer language, such as C, C++, JAVA, or a machine language, by which a processor of the computer may be read through a device interface of the computer, to execute the methods implemented by a program after the computer reads the program. The code may include a functional code related to a function that defines necessary functions that execute the methods, and the functions may include an execution procedure related control code necessary to execute the functions in its procedures by the processor of the computer. Further, the code may further include additional information that is necessary to execute the functions by the processor of the computer or a memory reference related code on at which location (address) of an internal or external memory of the computer should be referenced by the media. Further, when the processor of the computer is required to perform communication with another computer or server in a remote site to allow the processor of the computer to execute the functions, the code may further include a communication related code on how the processor of the computer executes communication with another computer or server or which information or medium should be transmitted and received during communication by using a communication module of the computer.

The inventive concept has the following effects.

First, because a sequence for maintaining a specific selection ratio is generated, a ratio between the number of extractions of the selection targets in the sequence of extractions may be made to be the same as or similar to a selection ratio as a selection target that has a large numerical value on the selection ratio appears frequently and a selection target that has a small numerical value on the selection ratio appears less. Accordingly, when the selection target is an activity (for example, weight training or a rehabilitation training exercise), the body portions of the user may be uniformly developed or rehabilitated by generating a sequence such that the performance ratio is suitable for the user.

Second, a sequence in which a plurality of selection targets are uniformly distributed is generated. Accordingly, when a plurality of activities or contents are provided, the interests of the user may be prevented from being lowered by preventing a specific activity or content from being repeatedly performed continuously.

While the inventive concept has been described with reference to embodiments, it will be apparent to those skilled in the art that various changes and modifications may be made without departing from the spirit and scope of the inventive concept. Therefore, it should be understood that the above embodiments are not limiting, but illustrative.

**Claims**

1. A method for determining a sequence of extractions by using a computer, the method comprising:

    a first selection ratio receiving operation of receiving a first selection ratio for a plurality of selection targets;
    a first extraction counter speed determining operation of determining speeds of first extraction counters for the selection targets, wherein the first extraction counters are set for the selection targets and wherein the speeds are proportional to the first selection ratio;
    a selection target extracting operation of, if a first extraction counter for a specific selection target reaches a reference value as the counter value is increased at a speed determined for the first extraction counter, extracting a selection target, the counter value of which reaches the reference value;
    a first extraction counter initializing operation of initializing the first extraction counter of the extracted selection target; and
    an operation of repeating the selection target extracting operation and the first extraction counter initializing operation.

2. A method for determining a sequence of extractions by using a computer, the method comprising:

    a first selection ratio receiving operation of receiving a first selection ratio for a plurality of selection targets;
    a first extraction counter speed determining operation of determining speeds of first extraction counters for the selection targets, wherein the first extraction counters are set for the selection targets and wherein the speeds are proportional to the first selection ratio;
    a selection target extracting operation of, as the counter values at speeds determined for the respective first extraction counters are increased, extracting a selection target, of which the counter value of the first extraction counter is maximal, from the plurality of selection targets;
a first extraction counter correcting operation of calculating a first calculation value by subtracting a sum of the speeds for the first extraction counters for the selection targets from the counter value of the selection target and applying the first calculation value as a corrected counter value; and
an operation of repeating the selection target extracting operation and the first extraction counter correcting operation.

3. The method of claim 1 or 2, wherein the first extraction counters correspond to clocks, and time change speeds of the clocks for the selection targets are applied differently according to the first selection ratio.

4. The method of claim 1 or 2, further comprising:
an operation of, when the plurality of selection targets are extracted at the same time point, determining the sequence of extractions at random.

5. The method of claim 1 or 2, further comprising:

    an operation of, when a least common multiple included in the terms of the first selection ratio is present, determining a correction value added to terms corresponding to the selection targets and calculating a final ratio added to the terms, wherein the correction value is a value that prohibits the terms in the final ratio have a least common multiple,
    wherein the first extraction counter speed determining operation includes:
    an operation of determining the speeds of the first extraction counters for the selection targets based on the final ratio.

6. The method of claim 5, wherein the correction value is a value between 0 and 1, which is determined for the selection targets by a random function.

7. The method of claim 1, wherein the selection target extracting operation and the repetition operation include:
an operation of extracting a specific selection target by increasing the counter value at a selection target extracting timing.

8. The method of claim 1, further comprising:

    when a plurality of detailed selection items are included in a selection target,
    a second selection ratio receiving operation of receiving a second selection ratio for the plurality of detailed selection items in the selection target, by the computer;

a second extraction counter speed determining operation of determining speeds of second extraction counters for the detailed selection items, wherein the second extraction counters are set for the detailed selection items and wherein the speeds are proportional to the second selection ratio;

a detailed selection item extracting operation of, if a second extraction counter for a specific detailed selection item reaches the reference value as the counter value is increased at a speed determined for the second extraction counter, extracting a detailed selection item, the counter value of which reaches the reference value; and

a second extraction counter initializing operation of initializing a counter of the extracted detailed selection item.

9. The method of claim 1, further comprising:
an operation of, when the selection target is a training type, calculating a first selection ratio for a plurality of training types.

10. The method claim 1, wherein the first selection ratio calculating operation includes:

an operation of acquiring current state data for a specific body portion of a specific user;

an operation of acquiring training level data by applying the current state data and normal state data to a training level calculation model;

an operation of calculating evaluation data for a specific training type by applying the acquired training level data to a training ratio determination model; and

an operation of determining the first selection ratio by calculating a ratio of evaluation data for a plurality of training types,

wherein the current state data is data for performance of a specific training type for a specific body portion of the user,

wherein the normal state data is data for performance of a specific training type for a specific body portion of a normal person, and

wherein the training level data is value data that represents a level by which performance of training according to the current state for a specific body portion of the user is allowed.

11. A program for determining a sequence of extractions by using a computer, which is coupled to a computer that is a piece of hardware and is stored in a medium to execute the method as claimed in any one of claims 1 to 10.

[FIG. 1]

Receive first selection ratio for plurality of selection targets by the computer [S200]

Determine speeds of first extraction counters for selection targets [S400]

Repeat process of extracting specific selection target from plurality of selection targets by driving first extraction counters for selection targets [S600]

[FIG. 2]

Receive first selection ratio for plurality of selection targets by the computer [S200]

Determine speeds of first extraction counters for selection targets [S400]

If first extraction counter for specific selection target reaches reference value as counter value increases at speed determined for first extraction counter, extract selection target counter value of which reaches reference value [S600]

Initialize first extraction counter of extracted selection target [611]

Repeating selection target extraction operation and first extraction counter initializing operation [612]

【FIG. 3】

Selection target A
First extraction counter

Selection target B
First extraction counter

Selection target C
First extraction counter

A   B   C

Initial setting

A   B   C

Performance result of
one-time extraction

[FIG. 4]

```
┌─────────────────────────────────────────────────────────────────┐
│   Receive first selection ratio for plurality of selection        │
│   targets by the computer [S200]                                  │
└─────────────────────────────────────────────────────────────────┘
                                │
                                ▼
┌─────────────────────────────────────────────────────────────────┐
│  Determine speeds of first extraction counters for selection      │
│  targets [400]                                                    │
└─────────────────────────────────────────────────────────────────┘
                                │
                                ▼
┌─────────────────────────────────────────────────────────────────┐
│  Extract selection target of which counter value of first         │
│  extraction counter is maximal, from plurality of selection       │
│  targets [S620]                                                   │
└─────────────────────────────────────────────────────────────────┘
                                │
                                ▼
┌─────────────────────────────────────────────────────────────────┐
│     Subtract sum of speeds for first extraction counters for      │
│  respective selection targets from counter value of selection     │
│  value and applying calculated first calculation value as         │
│  corrected counter value [622]                                    │
└─────────────────────────────────────────────────────────────────┘
                                │
                                ▼
┌─────────────────────────────────────────────────────────────────┐
│  Repeat selection target extracting operation and first           │
│  extraction counter correcting operation [624]                    │
└─────────────────────────────────────────────────────────────────┘
```

[FIG. 5]

EP 3 550 504 A1

[FIG. 6]

```
┌─────────────────────────────────────────────────────────────┐
│  Receive first selection ratio for plurality of selection    │
│              targets by the computer [S200]                  │
└─────────────────────────────────────────────────────────────┘
                              │
                              ▼
┌─────────────────────────────────────────────────────────────┐
│  Determine speeds of first extraction counters for selection │
│                       targets [400]                          │
└─────────────────────────────────────────────────────────────┘
                              │
                              ▼
┌─────────────────────────────────────────────────────────────┐
│  Repeat process of extracting specific selection target from │
│   plurality of selection targets by driving first extraction │
│            counters for selection targets [600]              │
└─────────────────────────────────────────────────────────────┘
                              │
                              ▼
┌─────────────────────────────────────────────────────────────┐
│  When plurality of selection targets are extracted at same   │
│   time point, determine sequence of extractions at random    │
│                         [S700]                               │
└─────────────────────────────────────────────────────────────┘
```

[FIG. 7]

```
┌─────────────────────────────────────────────────────┐
│  Receive first selection ratio for plurality of      │
│  selection targets by the computer [S200]            │
└─────────────────────────────────────────────────────┘
                          │
                          ▼
┌─────────────────────────────────────────────────────┐
│  When lease common multiple of terms included in     │
│  first selection ratio is present, calculate final   │
│  ratio added to terms by determining correction      │
│  value added to terms corresponding to selection     │
│  targets [300]                                        │
└─────────────────────────────────────────────────────┘
                          │
                          ▼
┌─────────────────────────────────────────────────────┐
│  Determine speeds of first extraction counters for   │
│  selection targets [S400]                            │
└─────────────────────────────────────────────────────┘
                          │
                          ▼
┌─────────────────────────────────────────────────────┐
│  Repeat process of extracting specific selection     │
│  target from plurality of selection targets by       │
│  driving first extraction counters for selection     │
│  targets [S600]                                       │
└─────────────────────────────────────────────────────┘
```

[FIG. 8]

```
┌─────────────────────────────────────────────────────┐
│  Receive first selection ratio for plurality of       │
│  selection targets by the computer [S200]             │
└─────────────────────────────────────────────────────┘
                          │
                          ▼
┌─────────────────────────────────────────────────────┐
│  Determine speeds of first extraction counters for    │
│  selection targets [S400]                             │
└─────────────────────────────────────────────────────┘
                          │
                          ▼
┌─────────────────────────────────────────────────────┐
│  Extract detailed selection item included in          │
│  extracted selection target [S500]                    │
└─────────────────────────────────────────────────────┘
                          │
                          ▼
┌─────────────────────────────────────────────────────┐
│  Repeat process of extracting specific selection      │
│  target from plurality of selection targets by        │
│  driving first extraction counters for selection      │
│  targets [[S600]                                      │
└─────────────────────────────────────────────────────┘
```

[FIG. 9]

EP 3 550 504 A1

Paint fence

Catch fish

Emergency call 119

Wrist
(bending/spreading out)

Wrist
(ulnar bending/radial bending)

Forearm
(pronation/supination)

Finger

[FIG. 10]

Receive first selection ratio for plurality of selection targets by the computer [S200]

↓

Determine speeds of first extraction counters for selection targets [S400]

↓

When plurality of detailed selection items are included in selection target, receive second selection ratio for plurality of detailed selection items in selection target by computer [S510]

↓

Determine speeds of second extraction counters for detailed selection items [S520]

↓

If second extraction counter for specific detailed selection item reaches reference value as counter value increases at speeds determined for second extraction counters [S530]

↓

Initialize counter of extracted selection target [S540]

↓

Repeat process of extracting specific selection target from plurality of selection targets by driving first extraction counters for selection targets [S600]

【FIG. 11】

Acquire current state data for specific body portion of specific user [S210]

↓

Acquire training level data by applying current state data and normal state data to training level calculation model [S220]

↓

Calculate evaluation data for specific training type by applying acquired training level data to training ratio determination model [S230]

↓

Determine first selection ratio by calculating ratio of evaluation data for plurality of training types [S240]

↓

Determine speeds of first extraction counters for selection targets [S400]

↓

Repeat process of extracting specific selection target from plurality of selection targets by driving first extraction counters for selection targets [[S600]

【FIG. 12】

Relative evaluation data

0.5

Normal state

0 Minimum state

Performance result data

[FIG. 13]

Training
level data
[Q]

$$Q = -3.125|x - 0.8| - 1.875x + 2.5$$

1

0      0.8 1 State evaluation
data [X]

## INTERNATIONAL SEARCH REPORT

| International application No. |
| --- |
| PCT/KR2017/011197 |

**A. CLASSIFICATION OF SUBJECT MATTER**

*G06Q 50/22(2012.01)i, G06Q 50/10(2012.01)i, A63B 24/00(2006.01)i*

According to International Patent Classification (IPC) or to both national classification and IPC

**B. FIELDS SEARCHED**

Minimum documentation searched (classification system followed by classification symbols)
G06Q 50/22; G06F 9/48; A63B 69/00; G01B 21/00; G06Q 50/00; A61B 5/00; G06F 9/50; G06Q 50/10; A63B 24/00

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: extraction, order, counter, speed, training, exercise

**C. DOCUMENTS CONSIDERED TO BE RELEVANT**

| Category* | Citation of document, with indication, where appropriate, of the relevant passages | Relevant to claim No. |
| --- | --- | --- |
| A | JP 4826743 B2 (KONICA MINOLTA MEDICAL & GRAPHIC INC.) 30 November 2011<br>See claims 1-2 and figure 2. | 1-11 |
| A | JP 2009-201681 A (XING INC. et al.) 10 September 2009<br>See paragraphs [0047], [0056]-[0057], claim 1 and figures 7, 16-17. | 1-11 |
| A | KR 10-1347692 B1 (FIT.LIFE INC.) 07 January 2014<br>See claims 1, 7 and figures 1-2. | 1-11 |
| A | JP 2004-184351 A (TOSHIBA CORP.) 02 July 2004<br>See claims 5-6 and figure 1. | 1-11 |
| A | JP 2012-038275 A (MIZUHO INFORMATION & RESEARCH INSTITUTE INC.)<br>23 February 2012<br>See paragraphs [0041]-[0050] and claims 1-2, 4, 9. | 1-11 |

☐ Further documents are listed in the continuation of Box C.   ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 29 JANUARY 2018 (29.01.2018) | **29 JANUARY 2018 (29.01.2018)** |
| Name and mailing address of the ISA/KR<br>Korean Intellectual Property Office<br>Government Complex-Daejeon, 189 Seonsa-ro, Daejeon 302-701,<br>Republic of Korea<br>Facsimile No. +82-42-481-8578 | Authorized officer<br><br>Telephone No. |

Form PCT/ISA/210 (second sheet) (January 2015)

INTERNATIONAL SEARCH REPORT
Information on patent family members

| Patent document cited in search report | Publication date | Patent family member | Publication date |
|---|---|---|---|
| JP 4826743 B2 | 30/11/2011 | JP 2007-193399 A<br>US 2007-0168231 A1<br>US 8301460 B2 | 02/08/2007<br>19/07/2007<br>30/10/2012 |
| JP 2009-201681 A | 10/09/2009 | JP 04854694 B2 | 18/01/2012 |
| KR 10-1347692 B1 | 07/01/2014 | US 2016-0256742 A1<br>WO 2014-092306 A1 | 08/09/2016<br>19/06/2014 |
| JP 2004-184351 A | 02/07/2004 | NONE | |
| JP 2012-038275 A | 23/02/2012 | NONE | |

Form PCT/ISA/210 (patent family annex) (January 2015)